# EUROPEAN PATENT SPECIFICATION

(11) **EP 2 173 297 B1**
(45) Date of publication and mention of the grant of the patent: **31.03.2021**
(21) Application number: 08796288.2
(22) Date of filing: 18.07.2008
(51) Int. Cl.: A61F 13/02, A61M 1/00, A61M 27/00, A61F 13/00, A61P 7/04, A61P 17/02

(54) **APPLICATION OF POLYMERIC MATERIALS TO SCREENS TO FACILITATE HEMOSTASIS AND WOUND HEALING**
ANWENDUNG VON POLYMERMATERIALIEN AUF GITTER FÜR VERBESSERTE HÄMOSTASE UND WUNDHEILUNG
APPLICATION DE MATÉRIAUX POLYMÈRES À DES TAMIS POUR FACILITER L'HÉMOSTASE ET LA CICATRISATION DE PLAIE

(30) Priority: 18.07.2007 US 959932 P
(43) Date of publication of application: 14.04.2010
(73) Proprietor: MARINE POLYMER TECHNOLOGIES, INC., Danvers, MA 01923 (US); The Brigham and Women's Hospital, Inc., Boston, MA 02199 (US)
(72) Inventor: ORGILL, Dennis, P., Belmont, MA 02478 (US); PIETRAMAGGIORI, Giorgio, Newton, MA 02459 (US); FINKIELSZTEIN, Sergio, Newton, MA 02459 (US); VOURNAKIS, John, N., Charleston, SC 29403 (US)
(74) Representative: Lock, Graham James
(86) International application number: PCT/US2008/070448
(87) International publication number: WO 2009/012438

(56) References cited:
- EP-A- 0 918 548
- EP-A2- 2 173 297
- WO-A-2005/123170
- WO-A-2007/016664
- US-A- 5 623 064
- US-A1- 2007 027 414
- US-A1- 2007 032 754

## Description

### FIELD OF THE INVENTION

This invention relates in general to a method and device for facilitating hemostasis and wound healing. In particular, the invention relates to the device comprising a polymeric material disposed on a scaffold that facilitates hemostasis and wound healing. Specifically, the invention contemplates the use of such scaffolds in conjunction with a negative pressure device.

### BACKGROUND OF THE INVENTION

WO 2005/123170 relates to wound dressings for vacuum therapy. The document discloses a dressing comprising a cover configured for placement over the wound to maintain a reduced pressure over the wound and adapted for communication with a source of vacuum, and a screen structure for placement between the cover and the wound, wherein the screen structure is adapted to remove or inactivate undesirable components from the wound environment and/or to concentrate desirable components present in the wound environment. The document also discloses systems comprising the wound dressings in combination with a source of vacuum.

US 2007/0027414 relates to laminar construction negative pressure wound dressing including bioabsorbable material. The document discloses a wound dressing system and method. The dressing is disposed in the wound in layers, including at least one bioabsorbable layer that contacts the wound bed, a bioabsorbable fluid communicating layer, an atmospheric barrier layer, and a tube for applying a negative pressure to the wound bed.

US 2007/0032754 relates to a method and apparatus for treating a wound. The document discloses applying a collagen material to the wound. In addition, the document discloses a method which includes overlying a wound dressing material upon the collagen material on the wound. The method also includes draining fluids from the wound through a tube. The tube has one end coupled to a vacuum source and another end positioned adjacent the wound. Negative pressure is applied to the wound through the tube.

While improvements in diagnostic tools and therapies have led to decreased morbidity from heart disease, cancer and stroke (Jemal, A., Ward, E., Hao, Y. & Thun, M. Trends in the leading causes of death in the United States, 1970-2002. Jama 294, 1255-9, 2005), the epidemic of diabetes (Gerstein, H.C. & Waltman, L. Why don't pigs get diabetes? Explanations for variations in diabetes susceptibility in human populations living in a diabetogenic environment. Cmaj 174, 25-6 2006) and the aging population (Lane, N.E. Epidemiology, etiology, and diagnosis of osteoporosis. Am J Obstet Gynecol 194, S3-11 2006) are now posing a critical challenge for wound care. (Cavanagh, P.R., Lipsky, B.A., Bradbury, A. W. & Botek, G. Treatment for diabetic foot ulcers. Lancet 366, 1725-35 2005); (Falanga, V. Wound healing and its impairment in the diabetic foot. Lancet 366, 1736-43 2005). Frequent in this population is the use of anticoagulants. Also many wounds in their inflammatory state can have significant bleeding even in the presence of normal clotting parameters. Equipment and techniques for accelerating wound healing are critical in the care of these patients. The availability of a device or system that would enhance both clotting and wound healing would be a significant advance in treatment.

Suction has long been a valuable tool in wound healing. The use of suction and related techniques in wound treatment has been well characterized in the literature. (See., e.g., Charikar and Jeter, Orringer, Wooding-Scott). Chest tubes, for example, re-approximate the parietal and visceral pleura while suction drains facilitate closure of large surgical spaces.

A recent improvement over suction alone in treating wounds has been the introduction of negative pressure or sub-atmospheric therapy systems as exemplified by the Vacuum Assisted Closure (VAC) systems of Argenta and Morykwas. (Argenta, L.C., Morykwas, M.J. Vacuum-assisted closure: a new method of wound control and treatment: clinical experience. Ann. Plast. Surg. 38: 563, 1997) ; (Morykwas, M.J., Argenta, L.C., Shelton-Brown, E.I., et al. Vacuum-assisted closure: a new method for wound control and treatment: animal studies and basic foundation. Ann. Plast. Surg. 38: 553, 1997); U.S. Patent No. 5,636,643; U.S. Patent No. 5,645, 081). Argenta et al. found that the controlled distribution of pressure throughout the wound is important in speeding wound healing. In the original design the negative pressure was distributed over a mesh applied directly to the wound site.

The VAC system has become the preferred method in many centers for treating a wide array of complex wounds. In its current commercial embodiment the VAC is a system comprising a vacuum pump that delivers sub-atmospheric pressure to a polyurethane ether open pore foam (400-600 µm) covered by an occlusive polyurethane drape. It includes an open pore polyurethane foam in contact with the wound site, a semi-occlusive drape, and a suction tube in addition to the vacuum or suction pump. Several prospective studies have shown that the VAC system increases the healing of chronic wounds at least twice as rapidly as conventional methods such as wet to dry dressing changes. (Joseph, E., Hamori, C.A., Bergman, S., et al. A prospective randomized trail of vacuum-assisted closure versus standard therapy of chronic nonhealing wounds. Wounds 12: 60, 2000); (Edington, M.T., Brown, K.R., Seabrook, B.R., et al. A prospective randomized evaluation of negative pressure wound dressing for diabetic foot wounds. Ann. Vase. Surg. 17: 645, 2003). Clinicians noted a rapid change in the wounds including overall shrinkage and induction of granulation tissue (Edington, M.T., Brown, K.R., Seabrook, B.R., et al. A prospective randomized evaluation of negative pressure wound dressing for diabetic foot wounds. Ann. Vasc. Surg. 17: 645, 2003); (Saxena, V., Hwang, C.W., Huang, S., et al. Vacuum-assisted closure: microdeformations of wounds and cell proliferation. Plast. Reconstr. Surg. 114: 1086, 2004).

Despite the commercial success of the device, it has certain limitations. One major limitation is that unless bleeding is completely stopped prior to use of the device, bleeding at the wound will continue or increase, often requiring removal of the VAC device. This has become particularly problematic given the increasing number of patients are on anticoagulants such as Coumadin, Heparin, Lovenox, Plavix and Aspirin. Having an effective method of obtaining hemostasis would be a great advantage to the VAC device in selected patients. Therefore a negative pressure wound device that incorporates hemostatic characteristics would be of great value to the wound care community.

There are many hemostatic agents currently on the market including micro-fibrillar collagen, oxidized regenerated cellulose, and lyophilized gelatin. Each of these agents can help with hemostasis, but in general, clinicians are reluctant to use these in many wounds because of the foreign body response that they can cause. Other methods such as fibrin glue are expensive and have at least a theoretical risk of viral transmission.

In addition, it would be preferable if the hemostatic agent used in such an application itself had a wound healing enhancing effect. Some hemostatic agents may provide control of hemorrhage and have a low foreign body response (as shown by favorable performance in an ISO implantation test) however it may have a negative wound healing effect. (E.g., Surgicel device manufactured by Ethicon, Inc.)

Therefore an appliance incorporating a hemostatic agent that could be incorporated into a negative pressure wound care device such as the VAC, that would also enhance (or at least not change) the efficacy of the device and would not induce significant foreign body response is highly desirable.

Highly homogeneous and pure poly-N-acetyl glucosamine (pGlcNAc) nanofibers can be isolated by the culture of a marine microalga. (Vournakis JN, Demcheva M, Whitson A, Guirca R, Pariser ER. Isolation, purification, and characterization of poly-N-acetyl glucosamine use as a hemostatic agent. J Trauma 2004, 57(1 Suppl):S2-6). pGlcNAc patches, which contain microalgal nanofibers (SyvekPatch™, Marine Polymer Technologies, Danvers, MA), have been characterized as hemostatic agents to control bleeding following catheter removal, and are currently used in interventional cardiology and radiology as non-invasive closure devices. (Voumakis JN, Demcheva M, Whitson A, Guirca R, Pariser ER. Isolation, purification, and characterization of poly-N-acetyl glucosamine use as a hemostatic agent. J. Trauma 2004,57(1 Suppl):S2-6); (Najjar SF, Healey NA, Healey CM, McGarry T, Khan B, Thatte HS, et al. Evaluation of poly-N-acetyl glucosamine as a hemostatic agent in patients undergoing cardiac catheterization: a double-blind, randomized study. J Trauma 2004;57(1 Suppl):S38-41 ).

The N-acetyl glucosamine-containing oligo- and polysaccharides are an important class of glycosaminoglycans, molecules largely represented in the dermis and have superior wound healing properties. They are already used for inhibition of surgical adhesions, relief from joint pain, and for skin replacement in reconstructive surgery. (Fazio VW, Cohen Z, Fleshman JW, van Goor H, Bauer JJ, Wolff BG, et al. Reduction in adhesive small-bowel obstruction by Seprafilm adhesion barrier after intestinal resection. Dis. Colon Rectum 2006;49(1):1-11); Pena Ede L, Sala S, Rovira JC, Schmidt RF, Belmonte C. Elastoviscous substances with analgesic effects on joint pain reduce stretch-activated ion channel activity in vitro. Pain 2002, 99(3):501-8); Orgill DP, Straus FH, 2nd, Lee RC. The use of collagen-GAG membranes in reconstructive surgery. Ann N YAcad. Sci. 1999;888:233-48; Pietramaggiori, G., Yang, H., Scherer, S.S., Kaipainen, A., Chan, R.K., Alperovich, M., Newalder, J., Demcheva, M., Vournakis, J.N., Valeri, R.C., Hechtman, H.B., Orgill, D.P. Effects of poly-N-acetyl glucosamine (pGlcNAc) patch on wound healing in db/db mouse. J. Trauma (2008) 64(3):803-808.; Vournakis, J., Eldridge, J., DemchevaM. and Muise-Helmericks, R. Poly-N-acetyl Glucosamine Nanofibers Regulate Endothelial Cell Movement and Angiogenesis: Dependency on Integrin Activation of Etsl. J. Vascular Res, (2008) 45:222-232.)

In addition, N-acetyl glucosamine is contained in chitosan, a polymer with demonstrated hemostatic properties. (Malette WG, Quigley HJ, Gaines RD, Johnson ND, Rainer WG. Chitosan: a new hemostatic. Ann Thorac Surg 1983, 36(1):55-8). Although based on similar molecules, pGlcNAc and chitosan have structural, chemical, and biological differences; the former is constituted of highly ordered insoluble fibers, while the latter demonstrates a heterogeneous and soluble structure. (Fischer TH, Connolly R, Thatte HS, Schwaitzberg SS. Comparison of structural and hemostatic properties of the poly-N-acetyl glucosamine Syvek Patch with products containing chitosan. Microsc Res Tech 2004, 63(3):168-74). These structural dissimilarities result in hemostatic differences between the two materials. When compared, pGlcNAc patches induced hemostasis in 100% of cases, whereas several chitosan-based patches performed worse than a gauze pad control. (Fischer TH, Connolly R, Thatte HS, Schwaitzberg SS. Comparison of structural and hemostatic properties of the poly-N-acetyl glucosamine Syvek Patch with products containing chitosan. Microsc Res Tech 2004, 63(3): 168-74).

Poly-N-acetyl glucosamine nanofibers interact with platelets, red blood cells and endothelial cells, (Thatte HS, Zagarins S, Khuri SF, Fischer TH. Mechanisms of poly-N-acetyl glucosamine polymer-mediated hemostasis: platelet interactions. J Trauma 2004;57(1 Suppl):S13-21); (Thatte HS, Zagarins SE, Amiji M, Khuri SF. Poly-N-acetyl glucosamine-mediated red blood cell interactions. J Trauma 2004;57(1 Suppl):S7-12) and accelerate hemostasis through a sequence of events that have been recently demonstrated. (Fischer TH, Thatte HS, Nichols TC, Bender-Neal DE, Bellinger AD, Voumakis JN. Synergistic platelet integrin signaling and factor XII activation in poly-N-acetyl glucosamine fiber-mediated hemostasis. Biomaterials 2005, 26(27):5433-43; Fischer, T.H., Valeri, C.R., Smith, C.J., Scull, C.M., Merricks, E.P., Nichols, T.P., Demcheva, M. and Vournakis, J.N. Non-classical Processes in Surface Hemostasis: Mechanisms for the Poly-N-acetyl glucosamine-induced Alteration of Red Blood Cell Morphology and Prothrombogenicity. (2008) J. Biomedical Materials Res, in press; Smith, C.J., Voumakis, J.N., Demcheva, M. and Fischer, T.H. Differential Effect of Materials for Surface Hemostasis on Red Blood Cel Morphology. (2008) Microscopic Res. Techniques, in press.)

Platelets specifically interact with the nanofibers of the pGlcNAc patch and, as a result, their activation is amplified. The activation response includes pseudopodia extension, shape change, integrin complex activation, activation of calcium signaling, phosphatidyl serine exposure on the surface membrane, binding of factor X to platelets and an acceleration of fibrin polymerization kinetics. Upon activation, platelets release vasoconstrictor substances and activate clotting after contact with the nanofibers, thus contributing to wound healing.

Therefore an appliance incorporating a hemostatic agent, preferably the hemostatic agent pGlcNac, that could be incorporated into a negative pressure wound care device such as the VAC would be highly desirable.

### DESCRIPTION OF THE FIGURES

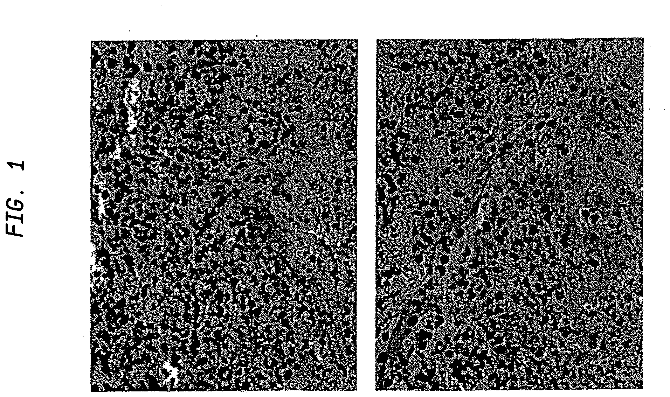

FIG. 1 shows wounds treated with pGlcNac and VAC technologies. 7 days post treatment wounds show high levels of proliferating cells contributing to wound healing (as shown by Ki-67 Immunohistochemical staining, a marker for actively proliferating cells).
FIG. 2 .shows a summary of the clinical observations of test animals of Example 1 indicating no signs of toxicity of the test articles.
FIG. 3 shows the results of a Macroscopic evaluation of the various test articles and controls articles implanted in Example 1.
FIG. 4A-C shows the results of the microscopic evaluation of the test articles and the control articles of Example 1 for each of the three test animals used.

### SUMMARY OF THE INVENTION

The invention is defined by the claims. The invention provides a system for delivering sub-atmospheric pressure to a wound for the purpose of wound healing and hemostasis that includes: a) a device that delivers a vacuum pressure in the range of 0 to 33.3 kPa (0 to 250 mm Hg); b) a seal that is semipermeable that covers the wound; c) a tube connecting the device to the seal; and d) a hemostatic appliance comprising: (i) a support that promotes uniform distribution of pressure within the wound and (ii) a hemostatic agent attached to the support; wherein the hemostatic agent is composed of poly-*N*-acetylglucosamine with a mean fiber size of about less than 10 microns. In one embodiment the support is a screen. In a further embodiment the support may be a foam.

In a preferred embodiment the fiber size is from about 2 to about 4 microns. The thickness of the wound healing appliance will vary according to the application and the type of support used. In one embodiment a thin film of poly-N-acelylglucosamine would act as its own scaffold and be absorbed into the wound site. In a different embodment the pGlcNac would be disposed over a wire mesh support.

In a further embodiment the pGlcNac would be disposed on or in a scaffold, preferably a porous scaffold such as a porous sponge that will allow cellular in-growth. The preparation of such porous sponges is well known in the art. Generally, the sponge would be prepared by lyophilization and would generally have a pore size of greater than 10 microns and less than 500 microns, preferably with pores in the range of 50-150 microns and most preferably with pore sizes about 100 microns.

### DETAILED DESCRIPTION OF THE INVENTION

### Background

It has been previously shown that shown that, in VAC systems, a foam, or screen, interface is critical for transferring subatmospheric pressure to the wound because the system causes microdeformations of wound tissue only in areas of foam contact (FC). (Saxena V, Hwang CW, Huang S, Eichbaum Q, Ingber D, Orgill DP. Vacuum -assisted closure: microdeformations of wounds and cell proliferation. Plast Reconstr Surg. 2004;1 14(5): 1086-96; discussion 1097-8). Wound areas covered with just a polyurethane drape without foam contact (WFC), in contrast, do not develop microdeformations although these areas are presumably exposed to at least some of the vacuum pressure. In addition, unlike WFC tissue, FC areas show significant granulation tissue. (Orgill DP, Bayer LR, Neuwalder J, Felter RC. Global surgery - future directions. Microdeformational wound therapy-a new era in wound healing. Business Briefing. 2005:22-25).

Although the VAC system and other suction devices are referred to as negative pressure wound therapy (NPWT) or sub-atmospheric wound therapy (SAWT), we prefer the term microdeformational wound therapy (MDWT) because a properly designed foam is required to transmit microdeformations to the wound surface. Several mechanisms may explain how MDWT accelerates wound closure. First, because cell shape is known to be important for cell proliferation, tension caused by microdeformations in the wound may activate signal transduction and cell division. (Armstrong, D.G. & Lavery, L.A. Negative pressure wound therapy after partial diabetic foot amputation: a multicentre, randomised controlled trial. Lancet 366, 1704- 10 2005); (Saxena, V. et al. Vacuum-assisted closure: microdeformations of wounds and cell proliferation. Plast Reconstr Surg 114, 1086-96; discussion 1097-8 2004); (Ulbrecht, J.S., Cavanagh, P.R. & Caputo, G.M. Foot problems in diabetes: an overview. Clin Infect Dis 39 Suppl 2, S73-82 2004); (Vournakis, J.N., Demcheva, M., Whitson, A., Guirca, R. & Pariser, E.R. Isolation, purification, and characterization of poly-N-acetyl glucosamine use as a hemostatic agent. J Trauma 57, S2-6 2004). Second, blood flow has been shown to increase as a result of MDWT in animals, although this has not been studied in humans. (Najjar, S.F. et al. Evaluation of poly-N-acetyl glucosamine as a hemostatic agent in patients undergoing cardiac catheterization: a double-blind, randomized study. J Trauma 57, S38-41 2004). Finally, the open pore foam may facilitate the removal of excess wound exudates, thus liminating harmful enzymes and improving nutrient diffusion.

### Device

The invention as defined in the claims, contemplates a wound healing system for delivering sub-atmospheric pressure to a wound for the purpose of wound healing and hemostasis that includes a) a device that delivers a vacuum pressure in the range of 0 to 33.3 kPa (0 to 250 mm Hg); b) a seal that is semipermeable that covers the wound; c) a tube connecting the device to the seal; and d) a hemostatic appliance comprising: (i) a support that promotes uniform distribution of pressure within the wound and (ii) a hemostatic agent attached to the support; wherein the hemostatic agent is composed of poly-*N*-acetylglucosamine with a mean fiber size of about less than 10 microns. In a preferred embodiment the appliance consists of an open pore polyurethane foam coated on its active surface with Poly-N-acetyl glucosamine nanofibers. The method of coating could include evaporation, lyophilization, casting or spraying.

Parameters that can be optimized during manufacture include the nature and concentration of various solvents, the thickness of the coating mechanism, the characteristics of the fibers (if any) of the hemostatic appliance coating, as well as the pH under which the device is manufactured.

In this invention the hemostatic or clotting agents can be used in sequence or as an integrated part of the scaffold. For example, in one example pGlcNac fibers were sprayed on traditional gauze wraps to attain hemostasis within a wound, prior to reapplying negative pressure wound therapy. More convenient to the surgeon, however would be to have the VAC device fabricated with the hemostatic agent already placed. Methods of fabrication are discussed below.

### Methods of Manufacture

One would appreciate that there are a number of different methods for manufacturing the hemostatic appliance of the invention. In particular, the appliance of the invention is comprised of an open cell or pore structure device coated or otherwise fabricated with pGlcNac. Methods include coating a support such a foam or a screen with the hemostatic agent by spraying or painting the agent on the support. Other fabrication methods can incorporate such techniques as microfabrication, lyophilization, the addition of the hemostatic agent with a microcarrier and nano-technology techniques.

### Examples

Numerous embodiments of the system and the device of the invention are contemplated. These include but are not limited to the device and systems shown in the examples below:

### Example 1: Biocompatability of Sample Implant

The purpose of the study was to evaluate the test article for the potential to induce local toxic effects after implantation in the muscle tissue of albino rabbits. The test article, MP719, (2-4 micron poly-N-acetyl glucosamine nanofibers; Marine Polymer Technologies, Inc., Danvers, MA), was implanted in the paravertebral muscle tissue of New Zealand White rabbits for a period of 4 weeks. The test article was evaluated separately using two control articles, Sponsor-specified Surgicel and Negative Control High Density Polyethylene (Negative Control Plastic). The results indicated that the test article was non-reactive when implanted for 4 weeks (Bioreactivity Rating of 0.2) when compared to Surgicel; and non-reactive (Bioreactivity Rating of 0.0) when compared to Negative Control High Density Polyethylene (Negative Control Plastic).

The study was conducted based upon the following references: ISO 10993-6, 1994, Biological Evaluation of Medical Devices - Part 6: Tests for Local Effects After Implantation; ISO 10993-12, 2002, Biological Evaluation of Medical Devices - Part 12: Sample Preparation and Reference Materials; ASTM F981-04, Standard Practice for Assessment of Compatibility of Biomaterials for Surgical Implants with Respect to Effect of Materials on Muscle and Bone, 2004; 2.4 ASTM F763-04, Standard Practice for Short Term Screening of Implant Materials, 2004; 2.5 ISO/IEC 17025, 2005, General Requirements for the Competence of Testing and Calibration Laboratories Methods and Materials

Three healthy New Zealand white rabbits (*Oryctolagus cuniculus)* 2 males and 1 female with a weight/age Range: 2.93 - 3.18 kilograms / at least 12 weeks old (adult) were used in the study

Albino rabbits were used in this study because they have historically been used in safety evaluation studies and the guidelines have no alternative (non-animal) methods. The species, number of animals, as well as the route of administration used, are recommended by the ISO 10993-6 guidelines.

The test article (MP719) measured approximately 1 mm to in width and 10 mm in length and was sterile. The two control articles were prepared. The first control, Surgicel (C1), measured approximately 1 mm in width by 10 mm in length and was received sterile. The second control, Negative Control Plastic (C2), measured approximately 1 mm in width by 10 mm in length and was sterilized by dipping in 70% ethanol.

Each animal was weighed prior to implantation. On the day of the test, the dorsal side of the animals were clipped free of fur and loose hair was removed by means of a vacuum. Each animal was appropriately anesthetized. Prior to implantation, the area was swabbed with a surgical preparation solution. Four test article strips were surgically implanted into each of the paravertebral muscles of each rabbit, approximately 2.5 cm from the midline and parallel to the spinal column and approximately 2.5 cm from each other. The test article strips were implanted on one side of the spine. In a similar fashion, control article strips (C1 - Surgicel) were implanted in the contralateral muscle of each animal. Two control strips (C2 - Negative Control Plastic) were implanted caudal (toward the tail) to the test article and to C1 control implant sites on either side of the spine (total of four strips). A total of at least eight test article strips and eight of each control article strips are required for evaluation.

The animals were maintained for a period of 4 weeks. The animals were observed daily for this period to ensure proper healing of the implant sites and for clinical signs of toxicity. Observations included all clinical manifestations. At the end of the observation period, the animals were weighed. Each animal was sacrificed by an injectable barbiturate. Sufficient time was allowed to elapse for the tissue to be cut without bleeding.

The paravertebral muscles in which the test or control articles were implanted were excised *in toto* from each animal. The muscle tissue was removed by carefully slicing around the implant sites with a scalpel and lifting out the tissue. The excised implant tissues were examined grossly, but without using excessive invasive procedures that might have disrupted the integrity of this tissue for histopathological evaluation. The tissues were placed in properly labeled containers containing 10% neutral buffered formalin. Following fixation in formalin, each of the implant sites was excised from the larger mass of tissue. The implant site, containing the implanted material, was examined macroscopically. Each site was examined for signs of inflammation, encapsulation, hemorrhaging, necrosis, and discoloration using the following scale: 0 = Normal; 1 = Mild; 2 = Moderate; 3 = Marked.

After macroscopic observation, the implant material was left *in-situ* and a slice of tissue containing the implant site was processed. Histologic slides of hematoxylin and eosin stained sections were prepared.

The following categories of biological reaction were assessed by microscopic observation for each implant site:
1. Inflammatory Responses:
   a. Polymorphonuclear leukocytes
   b. Lymphocytes
   c. Eosinophils
   d. Plasma cells
   e. Macrophages
   f. Giant cells
   g. Necrosis
   h. Degeneration
2. Healing Responses:
   a. Fibrosis
   b. Fatty Infiltrate

Each category of response was graded using the following scale: 0 = Normal; 0.5 = Very Slight; 1 = Mild; 2 = Moderate; 3 = Marked. The relative size of the involved area was scored by assessing the width of the area from the implant/tissue interface to unaffected areas which have the characteristics of normal tissue and normal vascularity. Relative size of the involved area was scored using the following scale:
0 = 0 mm, No site
0.5 = up to 0.5 mm, Very slight
1 = 0.6 - 1.0 mm, Mild
2 = 1.1 - 2.0 mm, Moderate
3 = > 2.0 mm, Marked

For each implanted site, a total score is determined. The average score of the test sites for each animal is compared to the average score of the control sites for that animal. The average difference between test and controls for all animals is calculated and the initial Bioreactivity Rating is assigned as follows:
0 - 1.5 No Reaction*
> 1.5 - 3.5 Mild Reaction
> 3.5 - 6.0 Moderate Reaction
> 6.0 Marked Reaction
* A negative calculation was reported as zero (0).

The pathology observer reviews the calculated level of bioreactivity. Based on the observation of all factors (e.g. relative size, pattern of response, inflammatory vs. resolution), the pathology observer revised the Bioreactivity Rating.

A descriptive narrative report regarding the biocompatibility of the test material is provided by the pathology observer. The study and its design employ methodology to minimize uncertainty of measurement and control of bias for data collection and analysis.

### Results.

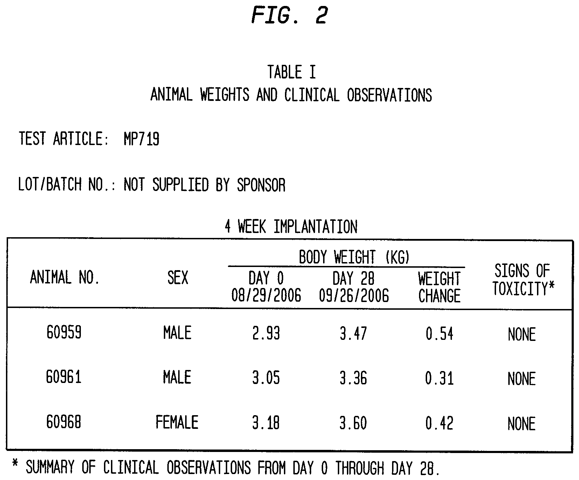

All three of the test animals increased in weight. None of the test animals exhibited any signs of toxicity over the course of the study. Clinical Observations (FIG. 2, Table I).

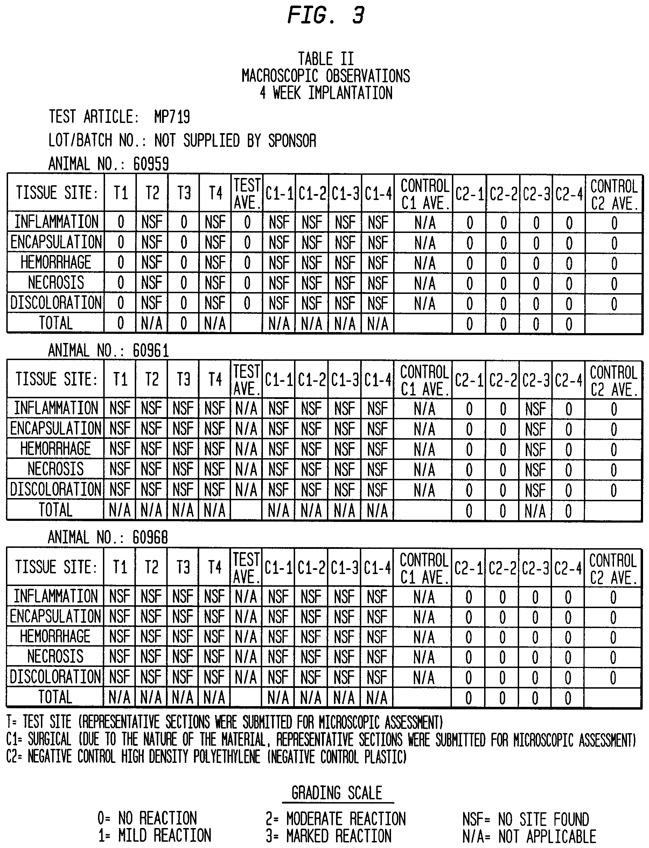

Macroscopic evaluation of the test article and control implant sites indicated no significant signs of inflammation, encapsulation, hemorrhage, necrosis, or discoloration at the 4 week time period. Some test sites and the majority of the Surgicel control, were not seen macroscopically and serial sections were submitted for microscopic evaluation. (FIG. 3, Table II).

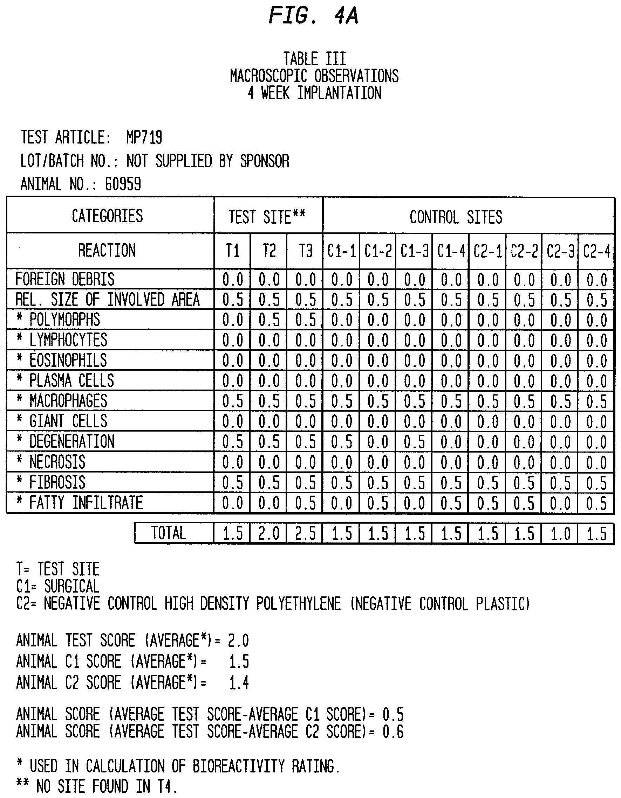

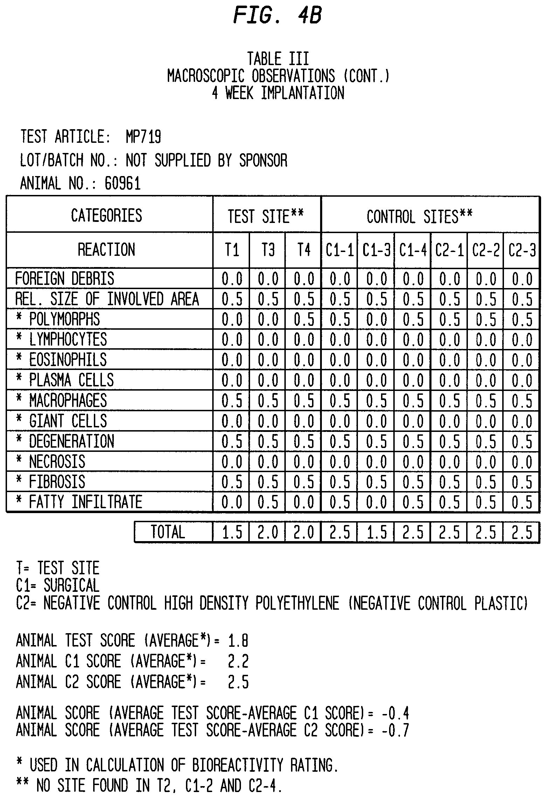

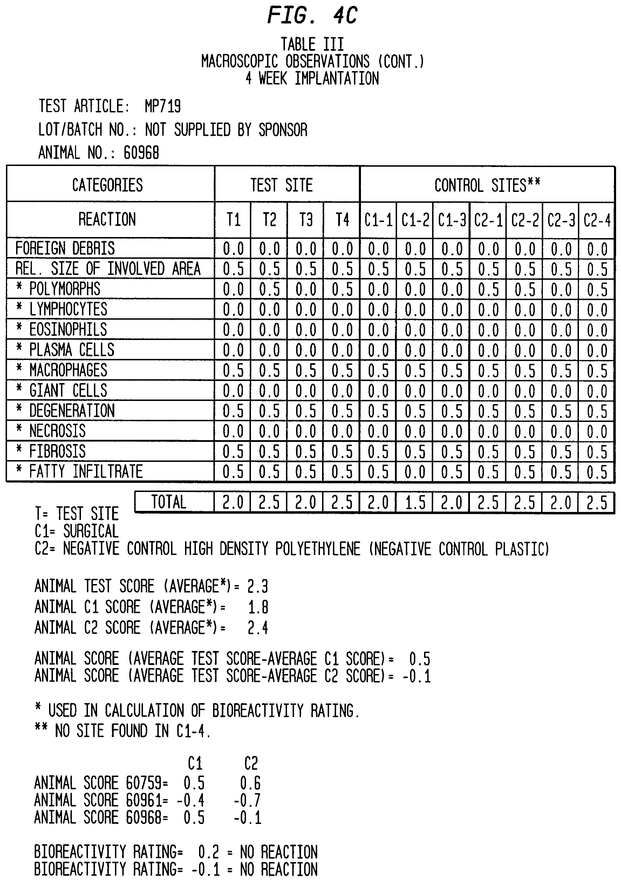

Microscopic evaluation of the test article implant sites indicated no significant signs of inflammation, fibrosis, hemorrhage, necrosis, or degeneration as compared to each of the control article sites. The Bioreactivity Rating for the 4 week time period (average of three animals) was 0.2, (C1 - Surgicel) and 0.0 (C2 - Negative Control Plastic) indicating no reaction as compared to either of the control implant sites. The pathologist noted there was a moderate polymorphic and histiocytic (macrophages) infiltrate around the *in situ* test article that was not unexpected given the nature of the test material (FIGs. 4A-C, Table III).

### Example 2: Diabetic Mouse - Granulation Tissue Measurements

Homozygous, genetically diabetic 8-12 week-old, Lep/r - db/db male mice (strain C57BL/KsJ-Lepr^{db}) were caged separately. Food and water were given ad libitum under an approved animal protocol in an AAALAC accredited facility. One day prior to surgery, dorsal hair was clipped and depilated (Nair®, Church & Dwight Co., Princeton, NJ). Animals were weighed and anesthetized with 60 mg/kg Nembutal (Pentobarbital) prior to surgery. The dorsum was disinfected with 70% alcohol and a 2 1.0 cm area of skin and panniculus carnosus was excised creating a full-thickness dorsal excisional wound. Wounds edges were protected with a 0.5 cm wide and 0.2 cm thick DuoDERM® (DuoDERM®, CGF®, ConvaTec, Squibb & Sons, L.L.C.) frame and dressing changes were performed on days 2 and 4. On day 7, animals were euthanized, and the wound area with its surrounding skin and underlying tissue was excised en block. The other half was fixed en block in 10% neutral-buffered formalin solution and kept in 70% alcohol at 4°C until paraffin embedment.

### Study Groups

Three animals were used in each study group. Granulation tissue responses were compared of wounds treated with:
1. Occlusive dressing (DuoDERM® frame) alone,
2. Complete VAC system (V.A.C., KCI, San Antonio, TX, 125 mm Hg suction),
3. Surgicel (Ethicon Inc., Somerville, NJ),
4. MP719 (2-4 micron poly-N-acetyl glucosamine nanofibers; Marine Polymer Technologies, Inc., Danvers, MA),
5. MP719 + VAC (125 mm Hg suction)

### Granulation Tissue Measurement

Paraffin embedded tissues were sectioned and stained according to routine Hematoxylin and Eosin (H&E) protocols. Panoramic sectional digital images of each H&E stained cross section wound were prepared using Adobe Photoshop CS Software (Adobe Systems Incorporated, San Jose, CA) to analyze granulation tissue area and thickness, using digital planimetry (Image J, NIH, Bethesda, MD) by two independent observers, blinded to the treatment, to quantify the area of granulation tissue in the middle part of each section at 10x magnification.

### Results

Data are shown in Table A below. The experimental groups, listed above, are compared to the Occlusive Dressing (OD) control. The data clearly show that the combination of MP719 and VAC provides an increase in the granulation tissue on day 7 of a wound healing study in the diabetic mouse animal model system. The combination of poly-N-acetyl glucosamine nanofibers plus VAC results in a greater than doubling of the granulation tissue generated at day 7. This is a strong indicator that the VAC - poly-N-acetyl glucosamine nanofibers combination provides a synergistic effect.

**TABLE A: Comparative Granulation Tissue Area (Day 7 data)**

| Sample | Number of Mice | % Granulation Tissue |
|---|---|---|
| OD | 3 | 100 |
| VAC | 3 | 140 |
| Surgicel | 3 | 90 |
| MP719 | 3 | 160 |
| MP719 + VAC | 3 | 330 |

### Example 3 - Hemostatic Effect & Synergy with Negative Pressure:

A 63-year-old female with hypertension, type 2 diabetes, and end-stage renal failure requiring dialysis (BMI 300 pounds and measuring 5 feet 4 inches) presented with a large mass along the lateral region of her left hip and thigh. Diagnosis at that time was undifferentiated sarcoma involving the left lateral soft tissues from the pelvis caudad to the iliac crest up to the distal one third of the thigh. The selected course of treatment involved external beam radiation followed by surgical resection.

Administering radiation therapy prior to surgical resection provides several potential benefits including reduced tumor volume and seeding during surgical manipulation and improved overall survival. However, the incidence of wound complications has been reported to be two-fold higher after preoperative compared with postoperative radiation therapy and such complications have been shown to have detrimental effects on patient function. Current guidelines from the National Comprehensive Cancer Network recommend an interval of 3 to 6 weeks between the end of preoperative radiation therapy and surgical resection to minimize risk of wound complications.

During the fifth week of radiation therapy severe bleeding from the sarcoma resulted in hemoglobin decrease to 5 gm/L. Radiation therapy was continued for the recommended total of 50.4 gray (Gy) over 5 weeks to complete the treatment cycle with the goal of ameliorating the bleeding. However, the patient had two additional bleeding episodes during the last week of radiation, each to the same low level of hemoglobin.

A CT scan of the thigh on showed multiple large vessels feeding the tumor along its entire base. Due to the uncontrolled bleeding and the patient's multiple co-morbidities, the multidiscipline medical team and the patient agreed to proceed with surgical resection at this time rather than wait the customary 3 to 6 weeks after radiation.

Surgical resection of an extremity soft tissue sarcoma immediately post radiation therapy is undesirable due to increased risk of thrombosis, bleeding, and complications of wound healing.

During end-block resection of the sarcoma and superficial groin dissection, prolific bleeding arose from the resection basin. The placement of 6 poly-N-Acteyl Glucosamine (pGlcNAc) hemostat pads along the resection basin between the left hip and thigh with pressure applied for 5 minutes brought immediate hemostasis to the surgical field.

The postsurgery wound was treated with negative pressure wound therapy (V.A.C.®, Kinetic Concepts Inc., San Antonio, Texas).

The combination of the pGlcNAc hemostatic pad and V.A.C. allowed for immediate application of the V.A.C. post surgically. The wound interface coated with pGlcNAc resulted in increased granulation tissue and accelerated preparation of the wound for a skin graft.

## Claims

1. A system for delivering sub-atmospheric pressure to a wound for the purpose of wound healing and hemostasis that includes:
a) a device that delivers a vacuum pressure in the range of 0 to 33.3 kPa (0 to 250 mm Hg);
b) a seal that is semipermeable that covers the wound;
c) a tube connecting the device to the seal; and
d) a hemostatic appliance comprising:
(i) a support that promotes uniform distribution of pressure within the wound and
(ii) a hemostatic agent attached to the support;
wherein the hemostatic agent is composed of poly-*N*-acetylglucosamine with a mean fiber size of less than 10 microns.

2. The system of claim 1, wherein the poly-*N*-acetylglucosamine has a mean fiber size of 2 to 4 microns.

3. The system of claim 2, wherein the support comprises a porous sponge.

4. The system of claim 3, wherein the sponge has a pore size of greater than 10 microns and less than 500 microns.

5. The system of claim 1, wherein the support is a screen.

6. The system of claim 1, wherein the support is a foam.

## Patentansprüche

1. System zum Verabreichen subatmosphärischer Drücke auf eine Wunde zum Zweck der Wundheilung und Hämostase, welches umfasst:
a) ein Gerät, das einen Unterdruck in dem Bereich von 0 bis 33,3 kPa (0 bis 250 mm Hg) liefert;
b) einen semipermeablen Verschluss, der die Wunde bedeckt;
c) eine Röhre, die das Gerät mit dem Verschluss verbindet; und
d) eine hämostatische Einrichtung, die umfasst:
(i) einen Träger, der eine gleichmäßige Verteilung des Druckes mit der Wunde fördert und
(ii) einen hämostatischen Wirkstoff, der auf die Unterlage aufgetragen ist;
worin der hämostatische Wirkstoff aus Poly-N-Acetylglukosamin mit einer durchschnittlichen Fasergröße von weniger als 10 Mikrometern besteht.

2. System nach Anspruch 1, worin das Poly-N-Acetylglukosamin eine durchschnittliche Fasergröße von 2 bis 4 Mikrometern aufweist.

3. System nach Anspruch 2, worin der Träger einen porösen Schwamm umfasst.

4. System nach Anspruch 3, worin der Schwamm eine Porengröße von größer als 10 Mikrometer und geringer als 500 Mikrometer aufweist.

5. System nach Anspruch 1, worin der Träger ein Maschengewebe ist.

6. System nach Anspruch 1, worin der Träger ein Schaum ist.

## Revendications

1. Système pour délivrer une pression inférieure à la pression atmosphérique à une plaie en vue de la cicatrisation de plaie et de l'hémostase qui inclut :
a) un dispositif qui délivre une pression négative dans la plage de 0 à 33,3 kPa (0 à 250 mm Hg) ;
b) un élément d'étanchéité qui est semi-perméable et qui couvre la plaie ;
c) un tube reliant le dispositif à l'élément d'étanchéité ; et
d) un appareil hémostatique comprenant :
(i) un support qui favorise une distribution uniforme de pression à l'intérieur de la plaie et
(ii) un agent hémostatique fixé au support ;
dans lequel l'agent hémostatique est composé de poly-*N*-acétylglucosamine avec une taille moyenne de fibre inférieure à 10 microns.

2. Système selon la revendication 1, dans lequel la poly-*N*-acétylglucosamine présente une taille moyenne de fibre de 2 à 4 microns.

3. Système selon la revendication 2, dans lequel le support comprend une éponge poreuse.

4. Système selon la revendication 3, dans lequel l'éponge présente une taille de pore supérieure à 10 microns et inférieure à 500 microns.

5. Système selon la revendication 1, dans lequel le support est un tamis.

6. Système selon la revendication 1, dans lequel le support est une mousse.
